# FASCICULE DE BREVET EUROPEEN

(11) **EP 0 530 286 B1**
(45) Date de publication et mention de la délivrance du brevet: **03.08.1994**
(21) Numéro de dépôt: 91910270.7
(22) Date de dépôt: 21.05.1991
(51) Int. Cl.: C07C 235/84, C07C 229/34, C07C 231/18, A61K 31/335

(54) **PROCEDE DE PREPARATION STEREOSELECTIVE DE DERIVES DE LA PHENYLISOSERINE**
VERFAHREN ZUR STEREOSELEKTIVEN HERSTELLUNG VON PHENYLISOSERINDERIVATEN
PROCESS FOR THE STEREOSELECTIVE PREPARATION OF PHENYLISOSERIN DERIVATIVES

(30) Priorité: 22.05.1990 FR 9006368
(43) Date de publication de la demande: 10.03.1993
(73) Titulaire: RHONE-POULENC RORER S.A., 92160 Antony (FR)
(72) Inventeur: CORREA, Arlène, 13560-Sao Carlos, SP (BR); DENIS, Jean-Noel Gîtes de Belledonne Apt. No. 3, F-38410 Uriage (FR); GREENE, Andrew-Elliot La Maison du Verger, F-38410 Uriage (FR)
(74) Mandataire: Pilard, Jacques
(86) Numéro de dépôt international: FR9100406
(87) Numéro de publication internationale: WO9117977

(56) Documents cités:
- EP-A- 0 253 738
- EP-A- 0 336 841
- EP-A- 0 414 610
- JOURNAL OF ORGANIC CHEMISTRY, vol. 51, no. 1, 1986, Easton, US; J.-N. DENIS et al: "An efficient, enantioselective synthesis of the taxol side chain", pages 46-50
- JOURNAL OF THE AMERICAN CHEMICAL SOCIETY, vol. 110, no. 17, 17 aout 1988, Gaston, PA, US; J.-N. DENIS et al: "A highly efficient practical approach to natural taxol", pages 5917-5919
- ARCHIV DER PHARMAZIE, vol. 308, 1975,Weinheim, DE; E. KAMANDI et al: "Die Synthese von beta-Phenylisoserinen durch Ammonolyse von beta-Phenyl-glycid-estern,II", pages 135-141

## Description

La présente invention concerne un procédé pour la préparation stéréosélective de dérivés de la phénylisosérine de formule générale : 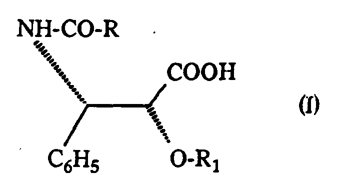
dans laquelle R représente un radical phényle ou un radical tert.butoxy et R₁ représente un groupement protecteur de la fonction hydroxy.

Dans la formule générale (I), R₁ représente plus particulièrement un radical méthoxyméthyle, éthoxy-1 éthyle, benzyloxyméthyle, (β-triméthylsilyléthoxy) méthyle, tétrahydropyrannyle ou trichloro-2,2,2 éthoxycarbonyle. De préférence, le radical R₁ est le radical éthoxy-1 éthyle.

Les produits de formule générale (I) sont utiles pour préparer les dérivés de la baccatine III et de la désacétyl-10 baccatine III de formule générale : 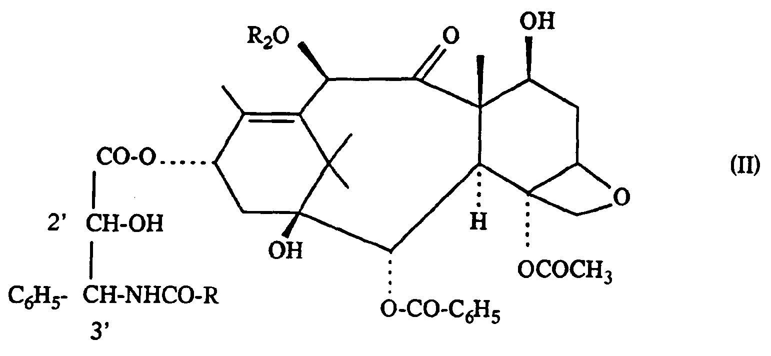
dans laquelle R représente un radical phényle ou un radical tert.butoxy et R₂ représente un atome d'hydrogène ou un radical acétyle.

Les produits de formule générale (II) dans laquelle R représente un radical phényle correspondent au taxol et au désacétyl-10 taxol et les produits de formule générale (II) dans laquelle R représente un radical tert.butoxy correspondent à ceux qui sont décrits dans le brevet européen EP 253 738.

Les produits de formule générale (II), et en particulier le produit de formule générale (II) dans laquelle R₂ représente un atome d'hydrogène et qui se présente sous la forme 2'R,3'S, présentent des propriétés antitumorales et antileucémiques particulièrement intéressantes.

Les produits de formule générale (II) peuvent être obtenus par action d'un produit de formule générale (I) sur un dérivé du taxane de formule générale : 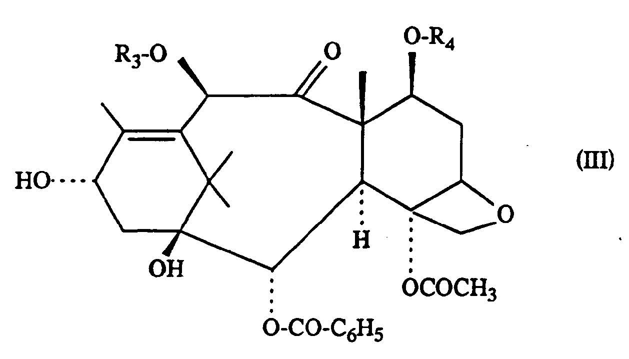
dans laquelle R₃ représente un radical acétyle ou un groupement protecteur de la fonction hydroxy et R₄ représente un groupement protecteur de la fonction hydroxy, suivie du remplacement des groupements protecteurs R₁ et R₄ et éventuellement R₃ par un atome d'hydrogène, dans les conditions décrites par J-N. DENIS et coll., J. Amer. Chem. Soc., 110 (17) 5917-5919 (1988).

Il est possible de faire réagir le produit racémique de formule générale (I) puis de séparer ultérieurement les diastéréoisomères du produit de formule générale (II) ou bien de faire réagir séparément chacun des énantiomères du produit de formule générale (I) sur le produit de formule générale (III).

Selon la présente invention, l'acide de formule générale (I) (forme syn, mélange racémique) peut être obtenu à partir de la benzylamine.

La benzylamine, par action d'un agent permettant d'introduire un groupement benzoyle ou t.butoxycarbonyle, est transformée en produit de formule générale : 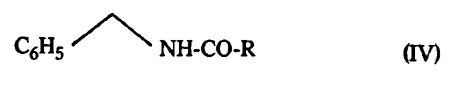
dans laquelle R est défini comme précédemment que l'on fait réagit, après double anionisation, avec l'acroléine pour donner l'alcool de formule générale : 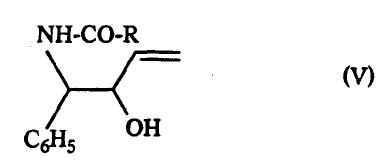
dans laquelle R est défini comme précédemment sous forme d'un mélange syn et anti contenant essentiellement la forme syn :

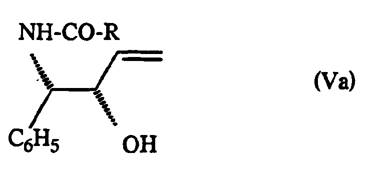

L'alcool de formule générale (Va), préalablement séparé du mélange des formes syn et anti, est oxydé en acide de formule générale (I) après protection de la fonction hydroxy.

Le produit de formule générale (IV) est généralement obtenu par action d'un agent permettant d'introduire un groupement benzoyle ou t.butoxycarbonyle, de préférence, selon le cas, le chlorure de benzoyle ou le dicarbonate de di-t.butyle. Généralement on opère dans un solvant organique tel que le chlorure de méthylène en présence d'une base minérale telle que la soude ou le bicarbonate ou le carbonate de sodium ou d'une base organique telle que la triéthylamine ou la diméthylamino-4 pyridine à une température comprise entre 0 et 50°C.

La double anionisation du produit de formule générale (IV) s'effectue généralement en utilisant 2 équivalents d'un dérivé organo-lithié tel que le s.butyllithium, en opérant dans un solvant organique anhydre tel que le tétra-hydrofuranne à une température inférieure à -50°C et de préférence voisine de -78°C.

Généralement la réaction de l'acroléine avec le dianion du produit de formule (IV) est effectuée en ajoutant l'acroléine, de préférence fraîchement distillée, à la solution du dianion préalablement refroidie au voisinage de -100°C. Après hydrolyse, on obtient le produit de formule générale (V) sous forme d'un mélange des diastéréoisomères syn et anti dont on sépare la forme syn de formule (Va) par chromatographie.

La protection de la fonction hydroxy de l'alcool de formule générale (Va) est effectuée dans les conditions habituelles de préparation des éthers et acétals, par exemple selon les procédés décrits par J-N. DENIS et coll., J. Org. Chem., 51, 46-50 (1986).

L'oxydation de l'alcool de formule générale (Va) protégé est de préférence réalisée au moyen d'un periodate alcalin (periodate de sodium) en présence d'une quantité catalytique d'un sel de ruthénium (RuCl₃) et de bicarbonate de sodium en opérant en milieu hydro-organique tel que par exemple un mélange tétrachlorure de carbone- acétonitrile-eau. Généralement la réaction est effectuée à une température voisine de 20°C.

L'oxydation peut aussi être réalisée au moyen de permanganate de potassium, par exemple en présence d'adogen dans un mélange pentane-eau ou en présence d'aliquat ou de dicyclohexyl-18 crown-6 dans le dichlorométhane ou dans le mélange pyridine-eau. Peut aussi être utilisé le permanganate de triéthylbenzylammonium en présence de pyridine dans le dichlorométhane.

Le produit de formule générale (I) (forme syn, mélange racémique) peut être dédoublé en ses énantiomères, et en particulier en son énantiomère 2R,3S, par exemple selon le procédé décrit par D. Petterson, Thèse de l'Université de Lund (Suède), pages 27-28 (1989).

Les exemples suivants, donnés à titre non limitatif, montrent comment l'invention peut être mise en pratique.

### EXEMPLE 1

Dans un monocol de 50 cm3 surmonté d'un réfrigérant et muni d'un système d'agitation magnétique, on introduit, sous argon, 218,5 µl (214,3 mg, 2 mmoles) de benzylamine et 10 cm3 de dichlorométhane sec. A la solution obtenue, on additionne 418 µl (303 mg, 3 mmoles) de triéthylamine et, par petites portions (réaction exothermique), 524 mg (2,4 mmoles) de dicarbonate de di-t.butyle pur. L'addition terminée, on laisse réagir pendant 4 heures à une température voisine de 20°C puis on dilue le mélange réactionnel résultant avec 40 cm3 de dichlorométhane. On lave la phase organique 4 fois avec 5 cm3 d'eau et 1 fois avec 5 cm3 d'une solution aqueuse saturée en chlorure de sodium. On sèche la phase organique sur sulfate de sodium anhydre. Après filtration, le dichlorométhane est éliminé sous pression réduite à l'évaporateur rotatif. Le résidu obtenu (505 mg) est purifié par chromatographie sur colonne de gel de silice en éluant avec un mélange acétate d'éthyle-chlorure de méthylène (5-95 en volumes). On obtient ainsi 406 mg (1,96 mmole) de benzylcarbamate de tbutyle sous forme d'un solide blanc avec un rendement de 98 % dont les caractéristiques sont les suivantes :
- point de fusion : 55,5-56,5°C (hexane)
- spectre infra-rouge (film) : bandes d'absorption caractéristiques à 3350, 3315, 3080, 3060, 3040, 3010, 2980, 2960, 2930, 1680, 1550, 1450, 1442, 1395, 1370, 1315, 1290, 1255, 1180, 1140, 1080, 1055, 1035, 950, 930, 918, 865, 770, 750, 725 et 700 cm⁻¹
- spectre de résonance magnétique nucléaire du proton (300 MHz ; CDCl₃ ; déplacements chimiques en ppm ; constantes de couplage J en Hz) : 1,46 (s, 9H); 4,3 (d, J = 5,7, 2H); 4,84 (s large, 1H) ; 7,22-7,34 (m, 5H)
- spectre de résonance magnétique nucléaire du ¹³C (CDCl₃) : 28,38 (CH₃) ; 44,69 (CH₂); 79,43 (C) ; 127,27 (CH) ; 127,41 (CH) ; 128,54 (CH) ; 138,93 (C) ; 155,84 (C).

### EXEMPLE 2

Dans un monocol de 250 cm3 mis sous argon et muni d'un système d'agitation magnétique, on introduit successivement 4,2 g (20,3 mmoles) de benzylcarbamate de t.butyle, 40 cm3 de tétrahydrofuranne anhydre et 6,5 cm3 (5,0 g, 43 mmoles) de tétraméthyléthylènediamine (TMEDA). On refroidit la solution obtenue à -78°C puis on lui additionne, goutte à goutte, 60 cm3 (60 mmoles) d'une solution de butyllithium secondaire 1M dans l'hexane. On laisse réagir pendant 3 heures à cette température puis on refroidit à -100°C. On ajoute alors 3 cm3 (2,5 g, 44,9 mmoles) d'acroléine fraîchement distillée, on laisse réagir pendant 3 à 4 minutes à cette température puis pendant 1 heure à -78°C. On hydrolyse le mélange réactionnel résultant à -78°C avec 20 cm3 d'eau, puis on l'extrait avec 2 fois 30 cm3 d'éther. Les phases organiques sont réunies puis elles sont lavées 2 fois avec 20.cm3 d'eau et avec 1 fois 10 cm3 d'une solution aqueuse saturée en chlorure de sodium. Elles sont ensuite séchées sur sulfate de sodium anhydre. Après filtration, les solvants sont éliminés sous pression réduite. Le résidu obtenu (11,6 g) est purifié sur colonne de gel de silice en éluant avec un mélange chlorure de méthylène-éther (95-5 en volumes). On obtient avec un rendement de 49 %, 2,606 g (9,91 mmoles) de phényl-1 t.butoxycarbonylamino-1 hydroxy-2 butène-3, sous forme du mélange des diastéréoisomères syn et anti dans le rapport 6/1.

Le diastéréoisomère syn est séparé du diastéréoisomère anti par chromatographie sur colonne de gel de silice en éluant avec un mélange éther-hexane-chlorure de méthylène (5-45-50 en volumes).

Le diastéréoisomère syn présente les caractéristiques suivantes :
- point de fusion : 86,5-88°C (hexane)
- spectre infra-rouge (film) : bandes d'absorption caractéristiques à 3400, 2975, 2920, 1690, 1500, 1450, 1390, 1365, 1250, 1175, 1080, 1050, 1020, 995, 920, 755 et 700 cm⁻¹
- spectre de résonance magnétique nucléaire du proton (300 MHz ; CDCl₃ : déplacements chimiques en ppm ; constantes de couplage J en Hz) : 1,40 (s, 9H); 1,9 (s large, 1H) ; 4,38 (pst, J = 4,6 et 4,8, 1H); 4,70 (s large, 1H) ; 5,20 (dt, J = 1,4 et 10,5, 1H) ; 5,26 (s large, 1H) ; 5,34 (dt, J = 1,4 et 17,2, 1H) ; 5,86 (ddd, J = 5,4, 10,5 et 17,2, 1H); 7,24-7,37 (m, 5H)
- spectre de résonance magnétique nucléaire du ¹³C (CDCl₃) : 28,12 (CH₃); 58,74 (CH) ; 75,33 (CH) ; 79,58 (C); 116,36 (CH₂); 126,69 (CH) ; 127,26 (CH) ; 128,32 (CH); 137,17 (CH) ; 139,96 (C) ; 155,89 (C)
- spectre de masse (i.c.) (NH₃ + isobutane) : 321 (M⁺ + isobutane) ; 281 (MH⁺ + NH₃) ; 264 (MH⁺, pic de base) ; 246, 225, 208, 190, 164, 124, 106
- analyse élémentaire :
   calculé % C 68,41 H 8,04 N 5,32
   mesuré % C 68,15 H 7,98 N 5,34

Le diastéréoisomère anti présente les caractéristiques suivantes :
- spectre infra-rouge (film) : bandes d'absorption caractéristiques à 3370, 3060, 2975, 2920, 1680, 1530, 1470, 1290, 1250, 1170, 1040, 1000, 930, 900, 870, 840, 755 et 700 cm⁻¹
- spectre de résonance magnétique nucléaire du proton (300 MHz ; CDCl₃ ; déplacements chimiques en ppm ; constantes de couplage J en Hz) : 1,41 (s, 9H); 1,8 (s large, 1H) ; 4,43 (psq, J = 0,9 et 4,4, 1H); 4,78 (s large, 1H); 5,18 (dt, J = 1,2 et 10,5, 1H) ; 5,24 (s large, 1H) ; 5,26 (dt, J =1,2 et 17, 1H) ; 5,71 (ddd, J = 5,5, 10,5 et 17, 1H); 7,24-7,36 (m, 5H)
- spectre de résonance magnétique nucléaire du ¹³C (CDCl₃) : 28,23 (CH₃); 59,22 (CH) ; 75,33 (CH) ; 79,85 (C) ; 117,06 (CH₂); 127,29 (CH) ; 127,56 (CH) ; 128,33 (CH); 136,27 (CH) ; 138,14 (C) ; 155,61 (C)
- analyse élémentaire :
   calculé % C 68,41 H 8,04 N 5,32
   mesuré % C 68,43 H 8,14 N 5,08

### EXEMPLE 3

Dans un monocol de 50 cm3 mis sous atmosphère d'argon et muni d'un système d'agitation magnétique, on introduit successivement 526 mg (2,0 mmoles) de phényl-1 t.butoxycarbonylamino-1 hydroxy-2 butène-3, forme syn, 20 cm3 de chlorure de méthylène sec, 1,9 cm3 (20,0 mmoles) d'éthylvinyléther distillé et 50,2 mg (0,2 mmole) de p-toluènesulfonate de pyridinium (PTSP). On laisse réagir le mélange réactionnel homogène résultant pendant 4,5 heures à une température voisine de 20°C. La réaction terminée, on ajoute 1 goutte de pyridine puis on dilue le mélange réactionnel dans 60 cm3 de chlorure de méthylène. On lave la phase organique 2 fois à l'eau, 2 fois avec une solution aqueuse saturée en chlorure de sodium puis on la sèche sur sulfate de sodium anhydre. Après filtration, les solvants sont éliminés sous pression réduite à l'évaporateur rotatif. On purifie le résidu obtenu par passage sur une colonne de gel de silice en éluant avec un mélange hexane-éther (8-2 en volumes). On obtient, avec un rendement de 87 %, 580 mg (1,73 mmole) de phényl-1 t.butoxycarbonylamino-1 (éthoxy-1 éthoxy)-2 butène-3 sous la forme de deux épimères dans le rapport 55/45 dont les caractéristiques sont les suivantes :
- point de fusion : 66-72°C
- spectre infra-rouge (film) : bandes d'absorption caractéristiques à 3370, 2970, 2925, 2875, 1680, 1520, 1495, 1365, 1285, 1250, 1170, 1080, 1050, 1005, 955, 930, 890, 870, 755 et 705 cm⁻¹
- spectre de résonance magnétique nucléaire du proton (300 MHz ; CDCl₃ ; déplacements chimiques en ppm ; constantes de couplage J en Hz) : 0,9 (min) et 1,07 (maj) (2t, J = 7, 3H); 1,05 (min) et 1,22 (maj) (2d, J = 5,3 (min) et 5,4 (maj), 3H); 1,40 (s, 9H); 2,90-2,98 et 3,05-3,51 (m, 2H); 4,16 et 4,23 (2psdd, J = 6,6 et 7, 1H) ; 4,31 (min) et 4,62 (maj) (2q, J = 5,3 (min) et 5,4 (maj), 1H) ; 4,71 (maj) et 4,73 (min) (2m, 1H) ; 5,22 et 5,23 (2dt, J = 1,2 et 10,5, 1H) ; 5,25 et 5,30 (2dt, J = 1,2 et 17,4, 1H) ; 5,37 et 5,44 (2m, 1H) ; 5,77 (min) et 5,91 (maj) (2ddd, J = 7, 10,5 et 17,4, 1H) ; 7,17-7,37 (m, 5H)
- analyse élémentaire :
   calculé % C 68,03 H 8,71 N 4,18
   mesuré % C 68,00 H 8,78 N 4,13

### EXEMPLE 4

Dans un monocol de 15 cm3 mis sous atmosphère d'argon et muni d'un système d'agitation magnétique, on met 251 mg (0,75 mmole) de phényl-1 t.butoxycarbonylamino-1 (éthoxy-1 éthoxy)-2 butène-3, forme syn en solution dans 1,5 cm3 d'acétonitrile. On ajoute ensuite successivement 1,5 cm3 de tétrachlorure de carbone, 2,25 cm3 d'eau distillée et, sous bonne agitation, 409,5 mg (4,875 mmoles) de bicarbonate de sodium. On ajoute ensuite, par petites portions, 882 mg (4,125 mmoles) de periodate de sodium. On laisse réagir le milieu réactionnel sous agitation pendant 5 minutes (dégagement gazeux) puis on additionne d'un coup 25,1 mg (10 % en poids) de RuCl₃. On laisse réagir, sous forte agitation, le mélange réactionnel devenu noir et fortement hétérogène pendant 48 heures à une température voisine de 20°C.

On dilue le mélange réactionnel avec de l'eau de façon à obtenir un volume total de 12 cm3. La phase aqueuse basique et noire est extraite 3 fois avec 20 cm3 d'éther. La phase basique est ensuite refroidie à 0°C, puis, en présence de 30 cm3 de chlorure de méthylène et sous forte agitation, elle est traitée, goutte à goutte, par 3 cm3 d'une solution aqueuse d'acide chlorhydrique 2M. La phase aqueuse acide résultante est extraite 8 fois avec 35 cm3 de chlorure de méthylène. Les phases organiques sont réunies et lavées avec 3 fois 8 cm3 d'eau et 1 fois 10 cm3 d'une solution aqueuse saturée en chlorure de sodium. Elles sont séchées sur un mélange 1/1 (p/p) de sulfate de sodium-sulfate de magnésium et filtrées sous pression réduite sur célite. Les solvants sont éliminés sous pression réduite jusqu'à un volume de 5 à 8 cm3. On sèche sur tamis moléculaire 4Å. On sépare la phase organique du tamis moléculaire puis le reste de solvant est éliminé à l'évaporateur rotatif.

On obtient avec un rendement de 77 %, 205 mg (0,58 mmole) d'acide phényl-3 t.butoxycarbonylamino-3 (éthoxy-1 éthoxy)-2 propionique syn pur sous forme d'une huile jaune pâle dont les caractéristiques sont les suivantes :
- spectre infra-rouge (film) : bandes d'absorption caractéristiques à 3700-2200, 3060, 2980, 2930, 2850, 1720, 1660, 1602, 1590, 1500, 1450, 1400, 1370, 1280, 1250, 1170, 1080, 1050, 1030, 955, 930, 890, 700 cm⁻¹
- spectre de résonance magnétique nucléaire du proton (300 MHz ; CDCl₃ ; déplacements chimiques en ppm ; constantes de couplage J en Hz) : 0,81 et 1,04 (2t, J = 7, 3H); 1,18 et 1,20 (2d, J = 5.4, 3H); 1,42 (s, 9H); 2,60-2,88 et 3,15-3,52 (m, 2H); 4,35-4,50 et 4,65-4,80 (m, 2H); 5,29 (s large, 1H) ; 5,72 (s large, 1H) ; 7,13-7,38 (m, 5H); 8,52 (s large, 1H).

## Revendications

1. Procédé de préparation stéréosélective de dérivés de la phénylisosérine de formule générale : 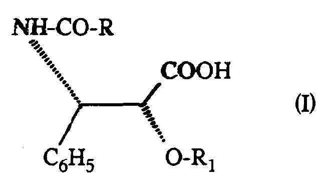 dans laquelle R représente un radical benzoyle ou tert.butoxy et R₁ représente un groupement protecteur de la fonction hydroxy, caractérisé en ce que l'on traite la benzylamine par un agent permettant d'introduire un groupement benzoyle ou t.butoxycarbonyle pour obtenir le produit de formule générale : 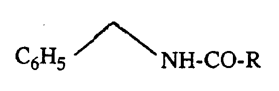 que l'on fait réagir, après double anionisation, avec l'acroléine pour donner l'alcool de formule générale : 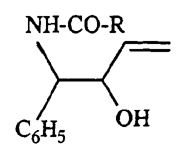 sous forme d'un mélange syn et anti dont on sépare la forme syn : 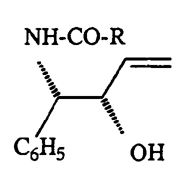 dont on protège la fonction hydroxy, puis oxyde l'alcool ainsi protégé pour obtenir le dérivé de la phénylisosérine de formule générale (I) sous forme syn dont on sépare éventuellement les énantiomères.

2. Procédé selon la revendication 1 caractérisé en ce que l'agent permettant d'introduire le groupement benzoyle ou t.butoxycarbonyle est le chlorure de benzoyle ou le dicarbonate de di-t.butyle que l'on fait réagir sur la benzylamine en opérant dans un solvant organique inerte tel que le chlorure de méthylène en présence d'une base minérale tel que la soude ou le bicarbonate ou le carbonate de sodium ou d'une base organique telle que la triéthylamine ou la diméthylamino-4 pyridine.

3. Procédé selon la revendication 1 caractérisé en ce que l'on fait réagir l'acroléine sur la N-benzylbenzamide ou le benzylcarbamate de t.butyl, préalablement anionisés en faisant réagir 2 équivalents d'un dérivé organo-lithié tel que le s.butyllithium, en opérant dans un solvant organique inerte tel que le tétrahydrofuranne à une température voisine de -100°C.

4. Procédé selon la revendication 1 caractérisé en ce que l'on protège la fonction hydroxy de l'alcool selon les méthodes habituelles puis oxyde l'alcool protégé au moyen d'un agent oxydant choisi parmi le periodate de sodium en présence d'une quantité catalytique d'un dérivé du ruthénium ou le permanganate de potassium.

5. Procédé de préparation de dérivés de la baccatine III et de la désacétyl-10 baccatine III de formule générale : 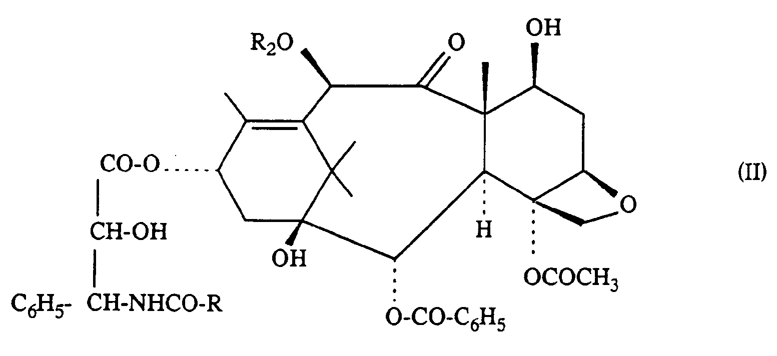 dans laquelle R représente un radical phényle ou un radical tert.butoxy et R₂ représente un atome d'hydrogène ou un radical acétyle, caractérisé en ce que l'on traite la benzylamine par un agent permettant d'introduire un groupement benzoyle ou tert.butoxycarbonyle pour obtenir le produit de formule générale : 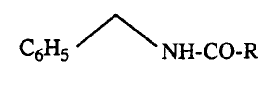 que l'on fait réagir, après double anionisation, avec l'acroléine pour donner l'alcool de formule générale : 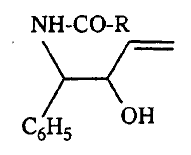 sous forme d'un mélange syn et anti dont on sépare la forme syn 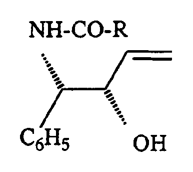 dont on protège la fonction hydroxy, puis oxyde l'alcool ainsi protégé pour obtenir le dérivé de la phénylisosérine de formule générale : 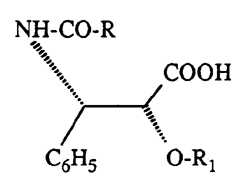 dans laquelle R₁ représente un groupement protecteur de la fonction hydroxy, sous forme syn, dont on sépare éventuellement les énantiomères, et que l'on fait réagir sur un dérivé du taxane de formule générale : 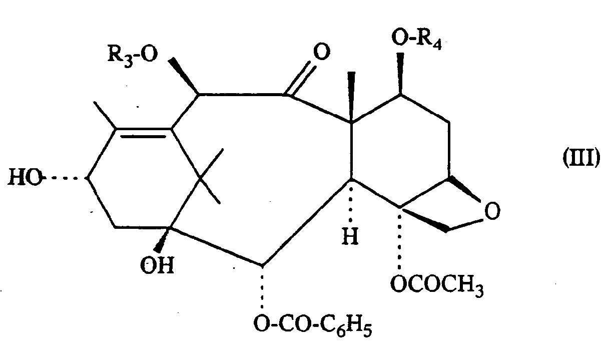 dans laquelle R₃ représente un radical acétyle ou un groupement protecteur de la fonction hydroxy et R₄ représente un groupement protecteur de la fonction hydroxy, puis on isole le produit de formule générale (II) après remplacement des groupements protecteurs R₃ et R₄ par des atomes d'hydrogène.

## Patentansprüche

1. Verfahren zur stereoselektiven Herstellung von Phenylisoserinderivaten der allgemeinen Formel 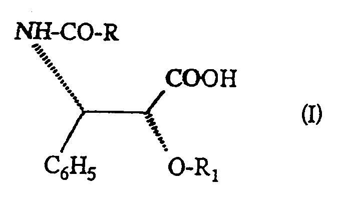 worin R für eine Benzoyl- oder tert.-Butoxygruppe steht, und R₁ für eine Schutzgruppe der Hydroxyfunktion steht, **dadurch gekennzeichnet,** daß man Benzylamin mit einem Mittel, das die Einführung einer Benzoyl- oder tert.-Butoxycarbonylgruppe erlaubt, behandelt, wodurch man die Verbindung der allgemeinen Formel 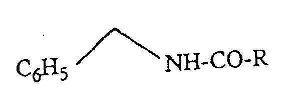 erhält, die man nach doppelter Anionisierung mit Acrolein umsetzt, wodurch man den Alkohol der allgemeinen Formel 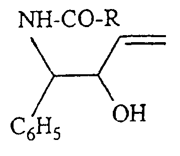 in Form eines Syn- und Anti-Gemisches erhält, wovon man die Syn-Form 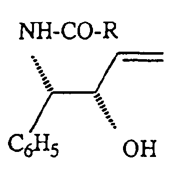 abtrennt, deren Hydroxyfunktion man schützt, anschließend den so geschützten Alkohol oxidiert, wodurch man das Phenylisoserinderivat der allgemeinen Formel (I) in der Syn-Form erhält, wovon man gegebenenfalls die Enantiomeren trennt.

2. Verfahren nach Anspruch 1, **dadurch gekennzeichnet**, daß das Mittel, das die Einführung der Benzoyl- oder tert.-Butoxycarbonylgruppe erlaubt, Benzoylchlorid oder Di-tert.-butyldicarbonat ist, das man mit Benzylamin in einem inerten organischen Lösungsmittel, wie Methylenchlorid, in Gegenwart einer mineralischen Base, wie Natriumhydroxid oder Natriumbicarbonat oder Natriumcarbonat, oder einer organischen Base, wie Triethylamin oder 4-Dimethylaminopyridin, umsetzt.

3. Verfahren nach Anspruch 1, **dadurch gekennzeichnet**, daß man Acrolein mit N-Benzylamid oder tert.-Butylbenzylcarbamat, das durch Umsetzung von 2 Äquivalenten eines Organolithiumderivates, wie sec.-Butyllithium, zuvor anionisiert worden ist, in einem inerten organischen Lösungsmittel, wie Tetrahydrofuran, bei einer Temperatur bei -100°C umsetzt.

4. Verfahren nach Anspruch 1, **dadurch gekennzeichnet**, daß man die Hydroxyfunktion des Alkohols nach an sich bekannten Verfahren schützt, anschließend den geschützten Alkohol mit einem Oxidationsmittel, ausgewählt aus Natriumperiodat in Gegenwart einer katalytischen Menge eines Rutheniumderivates oder Kaliumpermanganat, oxidiert.

5. Verfahren zur Herstellung von Baccatin-III- und 10-Desacetylbaccatin-III-Derivaten der allgemeinen Formel 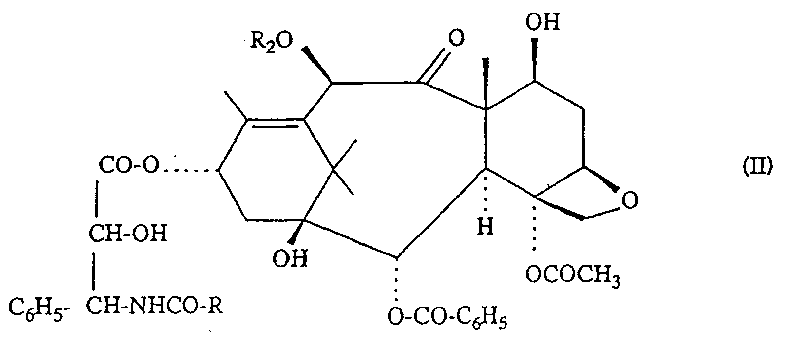 worin R für eine Phenyl- oder tert.-Butoxygruppe steht, und R₂ für ein Wasserstoffatom oder einen Acetylrest steht, **dadurch gekennzeichnet**, daß man Benzylamin mit einem Mittel, das die Einführung einer Benzoyl- oder tert.-Butoxycarbonylgruppe erlaubt, behandelt, wodurch man die Verbindung der allgemeinen Formel 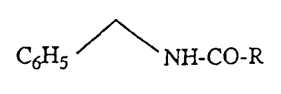 erhält, die man, nach doppelter Anionisierung, mit Acrolein umsetzt, wodurch man den Alkohol der allgemeinen Formel 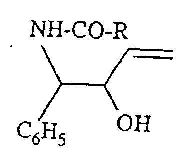 in Form eines Syn- und Anti-Gemisches erhält, wovon man die Syn-Form 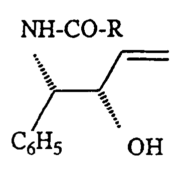 abtrennt, deren Hydroxyfunktion man schützt, anschließend den so geschützten Alkohol oxidiert, wodurch man das Phenylisoserinderivat der allgemeinen Formel 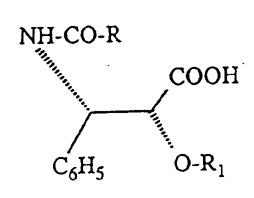 worin R₁ für eine Schutzgruppe der Hydroxyfunktion steht, in der Syn-Form erhält, wovon man gegebenenfalls die Enantiomeren trennt, und welches man mit einem Taxanderivat der allgemeinen Formel 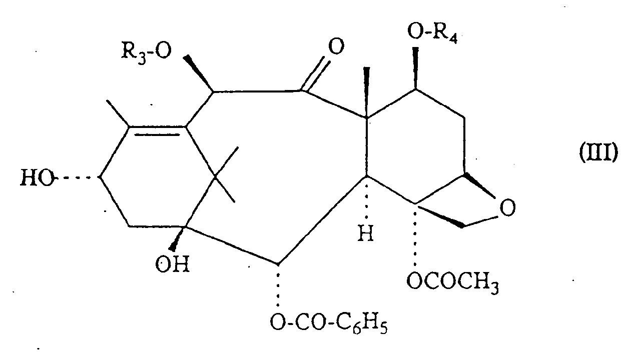 worin R₃ einen Acetylrest oder eine Schutzgruppe der Hydroxyfunktion bedeutet, und R₄ eine Schutzgruppe der Hydroxyfunktion bedeutet, umsetzt, anschließend die Verbindung der allgemeinen Formel (II) nach Ersatz der Schutzgruppen R₃ und R₄ durch Wasserstoffatome isoliert.

## Claims

1. Process for the stereoselective preparation of phenyl- isoserine derivatives of general formula 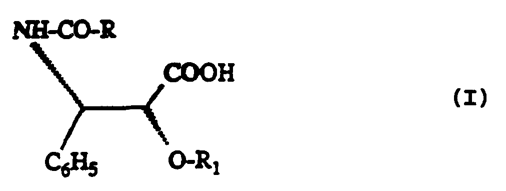 in which R is a benzoyl or tert-butoxy radical and R₁ is a protecting group for the hydroxyl group, characterized in that benzylamine is treated with an agent for introducing a benzoyl or t-butoxycarbonyl group to give the product of general formula 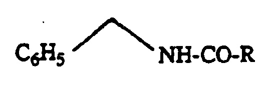 which, after double anionization, is reacted with acrolein to give the alcohol of general formula 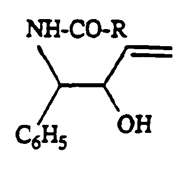 in the form of a syn and anti mixture, from which the syn form: 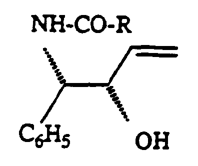 is separated, the hydroxyl group of said syn form is protected and the alcohol protected in this way is then oxidized to give the phenylisoserine derivative of general formula (I) in the syn form, which is separated into the enantiomers if appropriate.

2. Process according to Claim 1, characterized in that the agent for introducing the benzoyl or t-butoxycarbonyl group is benzoyl chloride or di-t-butyl dicarbonate, which is reacted with benzylamine in an inert organic solvent such as methylene chloride, in the presence of an inorganic base such as sodium hydroxide or sodium bicarbonate or carbonate, or an organic base such as triethylamine or 4-dimethylaminopyridine.

3. Process according to Claim 1, characterized in that acrolein is reacted with N-benzylbenzamide or t-butyl benzylcarbamate, previously anionized by reaction with 2 equivalents of an organolithium derivative such as s-butyllithium, the operation being carried out in an inert organic solvent such as tetrahydrofuran, at a temperature of about -100°C.

4. Process according to Claim 1, characterized in that the hydroxyl group of the alcohol is protected in accordance with the normal methods and the protected alcohol is then oxidized by means of an oxidizing agent selected from sodium periodate in the presence of a catalytic amount of a ruthenium derivative, or potassium permanganate.

5. Process for the preparation of derivatives of baccatine III and of 10-deacetylbaccatine III of general formula: 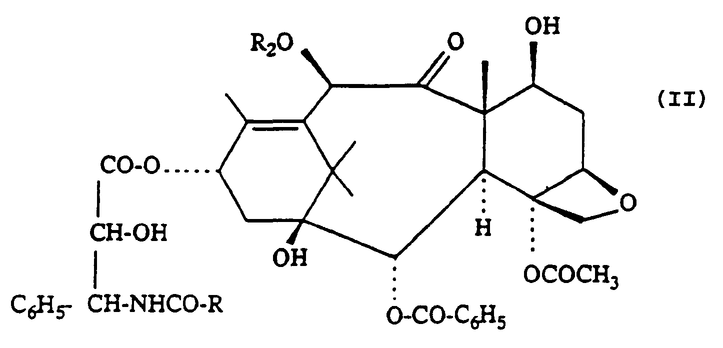 in which R is a phenyl radical or a tert-butoxy radical and R₂ is a hydrogen atom or an acetyl radical, characterized in that benzylamine is treated with an agent for introducing a benzoyl or tert-butoxycarbonyl group to give the product of general formula 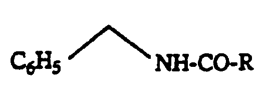 which, after double anionization, is reacted with acrolein to give the alcohol of general formula: 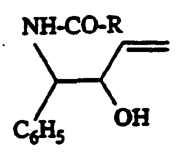 in the form of a syn and anti mixture, from which the syn form: 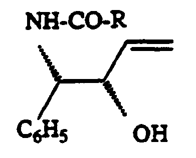 is separated, the hydroxyl group of said syn form is protected and the alcohol protected in this way is then oxidized to give the phenylisoserine derivative of general formula: 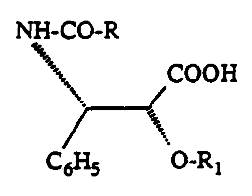 in which R₁ is a group for protecting the hydroxyl group, in syn form, which is separated into the enantiomers if appropriate, and which is reacted with a taxane derivative of general formula: 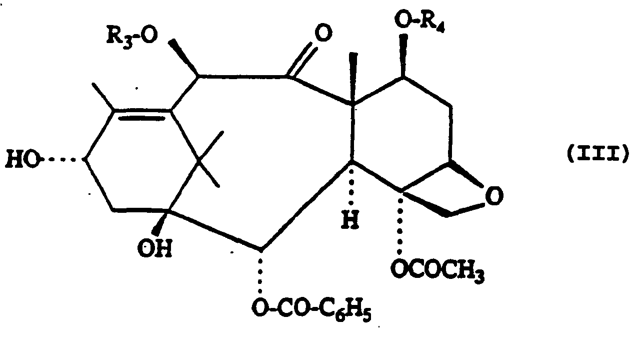 in which R₃ is an acetyl radical or a group for protecting the hydroxyl group and R₄ is a group for protecting the hydroxyl group, and the product of general formula (II) is then isolated after replacement of the protective groups R₃ and R₄ with hydrogen atoms.
